# EUROPEAN PATENT APPLICATION

(11) **EP 4 582 435 A1**
(43) Date of publication of application: **09.07.2025**
(21) Application number: 23859820.5
(22) Date of filing: 29.06.2023
(51) Int. Cl.: C07K 7/08, C07K 1/113, C07K 1/13, C07K 19/00, C12N 1/15, C12N 1/19, C12N 5/10, C12N 15/11, C12N 15/54, C12N 15/87

(54) **CARRIER PEPTIDE FRAGMENT AND USE THEREOF**

(30) Priority: 30.08.2022 JP 2022137043
(71) Applicant: Toagosei Co., Ltd., Minato-ku Tokyo 105-8419 (JP)
(72) Inventor: BAILEYKOBAYASHI, Nahoko, Tsukuba-shi, Ibaraki 300-2611 (JP); YOSHIDA, Tetsuhiko, Tsukuba-shi, Ibaraki 300-2611 (JP)
(74) Representative: Bals & Vogel Patentanwälte PartGmbB
(86) International application number: PCT/JP2023/024103
(87) International publication number: WO 2024/048050

(57) **Abstract**

The present disclosure provides a carrier peptide fragment with excellent cell membrane permeability. The carrier peptide fragment disclosed herein includes the following amino acid sequence set forth in SEQ ID No.: 1: KKRTLRKSNRKKR, and a lysine residue at a 12th position from an N-terminus of the amino acid sequence is a D-form, and the other amino acid residues are L-forms.

## Description

### [Technical Field]

The present invention relates to a method for transferring (carrying) a foreign substance from outside a eukaryotic cell into the cell, and a carrier peptide fragment used in the method. The present application claims priority on the basis of Japanese Patent Application No. 2022-137043 filed on August 30, 2022, and the entire content of this application is incorporated into the description of the present application by reference.

### [Background Art]

Conventionally, polypeptides and other foreign substances, particularly biologically active substances, have been transferred into the cells of humans and other mammals, etc., (eukaryotic cells) to change the characteristics of the cells (as well as the tissues and organs including the cells) or to improve and enhance the function of the cells.

For example, Patent Literature 1 discloses a construct including a carrier peptide fragment including an amino acid sequence: KKRTLRKSNRKKR (SEQ ID No.: 2), and a foreign substance. This carrier peptide fragment is a cell-penetrating peptide (hereinafter also referred to as "CPP") that has excellent cell membrane permeability; therefore, the construct including the carrier peptide fragment is efficiently transferred from outside a eukaryotic cell to the inside.

### [Citation List]

### [Patent Literature]

[Patent Literature 1] Japanese patent application publication No. 2022-047613

### [Summary of Invention]

For example, from the viewpoints of medical science and the like, it has been examined to use the CPP for a drug delivery system and the like. Thus, it has been desired to develop the CPP with the excellent cell membrane permeability.

The art disclosed herein has been made in view of the above circumstances, and a main object of the present disclosure is to provide a carrier peptide fragment with excellent cell membrane permeability. It is another object of the present disclosure to provide a construct for transferring a foreign substance including such a carrier peptide fragment. It is still another object of the present disclosure to provide a method for transferring a target foreign substance from outside a eukaryotic cell into at least a cytoplasm of the cell.

In order to create a carrier peptide fragment that exhibits more excellent cell membrane permeability on the basis of the amino acid sequence (SEQ ID No.: 2) disclosed in Patent Literature 1, the present inventors conducted earnest examinations. As a result, it has been found that when one of amino acid residues in the amino acid sequence set forth in SEQ ID No.: 2 is a D-form amino acid residue, the cell membrane permeability is improved. In particular, it has been found that when only a lysine residue at a 12th position from an N-terminus of the amino acid sequence set forth in SEQ ID No.: 2 (also referred to as lysine residue) is a D-form, especially excellent cell membrane permeability is achieved.

That is to say, one aspect of the carrier peptide fragment disclosed herein is a carrier peptide fragment to be transferred from outside a eukaryotic cell into at least a cytoplasm of the cell. This carrier peptide fragment includes the following amino acid sequence set forth in SEQ ID No.: 1: KKRTLRKSNRKKR, and a lysine residue at a 12th position from an N-terminus of the amino acid sequence is a D-form, and the other amino acid residues are L-forms. With such a structure, the excellent cell membrane permeability is achieved.

Moreover, the present disclosure provides a construct for transferring a foreign substance including the carrier peptide fragment disclosed herein (hereinafter also referred to as "construct" simply). One aspect of the construct disclosed herein is a construct for transferring a foreign substance manufactured to transfer a target foreign substance from outside a eukaryotic cell into at least a cytoplasm of the cell, and includes the carrier peptide fragment disclosed herein and a foreign substance that is bonded to an N-terminus and/or a C-terminus of the carrier peptide fragment. With such a structure, a construct that exhibits excellent cell membrane permeability is achieved.

In one aspect of the construct disclosed herein, the foreign substance can be at least one kind of organic compound selected from the group consisting of a polypeptide, a nucleic acid, a dye, and a drug.

Here, the term "polypeptide" refers to a polymer with a structure in which a plurality of amino acids is linked by peptide bonds. The polypeptide is not limited by the number of peptide bonds (that is, the number of amino acid residues). In other words, the term "polypeptide" encompasses those generally called peptides with about 10 or more and less than 300 amino acid residues and those generally called proteins (typically, high molecular compounds with 300 or more amino acid residues). In this field, polypeptides and proteins are not distinguished strictly. In this specification, polymers formed of a plurality of amino acid residues (including oligomers) are collectively referred to as polypeptides.

Moreover, the term "nucleic acid" refers to a nucleotide polymer and includes DNA and RNA. The term "nucleic acid" is not limited by the number of bases.

In one aspect of the construct disclosed herein, the foreign substance can be disposed on the C-terminus of the carrier peptide fragment.

Moreover, the present disclosure provides a method for transferring a target foreign substance in vitro or in vivo from outside a eukaryotic cell into at least a cytoplasm of the cell. The method disclosed herein includes the steps of: (1) preparing a construct disclosed herein; and (2) supplying the construct into a sample including a target eukaryotic cell. Thus, the target foreign substance can be transferred efficiently into the eukaryotic cell.

In one aspect of the method disclosed herein, the eukaryotic cell to which the construct is to be transferred can be a human or nonhuman mammalian cell.

### [Brief Description of Drawings]

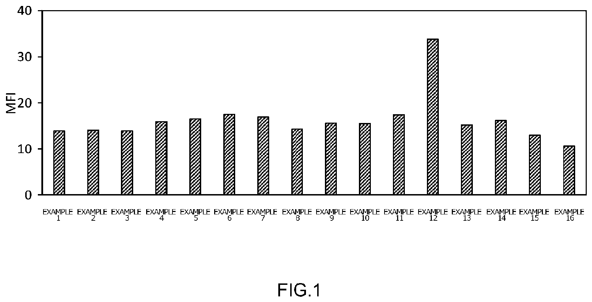

Fig. 1 is a graph illustrating values of MFI obtained by culturing with constructs (additives) shown in Examples 1 to 16 added to culture solutions of HeLa cells, and then analyzing the cells with a flow cytometer.

### [Description of Embodiments]

Some embodiments of the art disclosed herein will be described below. Matters that are other than matters particularly mentioned in the present specification and that are necessary for the implementation of the present art (for example, general matters related to chemical synthesis methods for peptide, cell culture techniques, and preparation of constructs containing peptides or nucleic acids as a component) can be grasped as design matters of those skilled in the art based on the prior art in the fields such as cell engineering, physiology, medical science, pharmacology, organic chemistry, biochemistry, genetic engineering, protein technology, molecular biology, and genetics.

Moreover, the art disclosed herein can be carried out on the basis of the contents disclosed in this specification and common technical knowledge in the fields. In the following description, the amino acids may be described by single letter codes based on the nomenclature for amino acids in the IUPAC-IUB guidelines. Note that the term "amino acid residue" in this specification encompasses N-terminus amino acids and C-terminus amino acids of a peptide chain unless otherwise stated specifically.

In this specification, the term "synthetic peptide" refers to a peptide fragment whose peptide chain alone does not stably exist in nature. The synthetic peptide is manufactured by artificial chemical synthesis or biosynthesis (that is, production based on genetic engineering) and can stably exist in a predetermined composition. Here, the term "peptide" refers to an amino acid polymer having a plurality of peptide bonds and is not limited by the number of amino acid residues.

The amino acid residue of the peptide or protein in this specification may be either an L-form or a D-form unless otherwise stated in particular. However, the L-form and the D-form of an amino acid sequence shown in a sequence listing are distinguished.

Note that the amino acid sequence in this specification always represents the N-terminus on the left side and the C-terminus on the right side.

One aspect of a carrier peptide fragment disclosed herein includes an amino acid sequence set forth in SEQ ID No.: 1: KKRTLRKSNRKKR. In this amino acid sequence, a lysine residue at a 12th position from the N-terminus is a D-form amino acid (that is, D-lysine residue), and the other amino acid residues (amino acid residues at 1st to 11th, and 13th positions from the N-terminus) are formed of L-form amino acids.

The carrier peptide fragment disclosed herein may be a modified sequence of the amino acid sequence set forth in SEQ ID No.: 1 unless the cell membrane permeability is deteriorated. Here, the "modified sequence" corresponds to an amino acid sequence (modified amino acid sequence) formed by the substitution, deletion, and/or addition (insertion) of one or several (typically, two or three) amino acid residues.

Typical examples of the modified sequence in this specification include a sequence produced by so-called conservative amino acid replacement in which one, two, or three amino acid residues are conservatively replaced, a sequence in which one, two, or three amino acid residues are added (inserted) to or deleted from a predetermined amino acid sequence, and the like. Typical examples of the conservative amino acid replacement include a sequence in which a basic amino acid residue is replaced by another basic amino acid residue (for example, mutual replacement between a lysine residue and an arginine residue), a sequence in which a hydrophobic amino acid residue is replaced by another hydrophobic amino acid residue (for example, mutual replacement of a leucine residue, an isoleucine residue, and a valine residue), and the like.

The construct for transferring a foreign substance disclosed herein includes the carrier peptide fragment disclosed herein, and a foreign substance that is bonded to an N-terminus and/or a C-terminus of the carrier peptide fragment.

The construct disclosed herein can be designed and configured by bonding (linking), either directly or indirectly via a suitable linker, a desired foreign substance to the N-terminus and/or the C-terminus of the abovementioned carrier peptide fragment.

The linker is not limited in particular and may be either a peptidic linker or a non-peptidic linker. Although there is no particular limitation, the amino acid sequence of the peptidic linker is preferably a flexible amino acid sequence that does not cause steric hindrance. Examples of the possible peptidic linker include linkers including ten or less (more preferably one or more and five or less, for example one, two, three, four, or five amino acid residues) amino acid residues including one kind or two or more kinds of amino acid residues selected from glycine, alanine, serine, and the like. As such a linker, β-alanine may be used. As the non-peptidic linker, for example, an alkyl linker, a polyethylene glycol (PEG) linker, an amino hexanoyl spacer, or the like can be used, without particular limitations.

The foreign substance can be, for example, an organic compound such as a polypeptide, a nucleic acid, a dye, or a drug.

If the foreign substance is a polypeptide, the target construct for transferring a foreign substance can be manufactured in such a way that a peptide chain is designed to contain the amino acid sequence constituting the polypeptide and the amino acid sequence constituting the carrier peptide fragment, and then the peptide chain is subjected to biosynthesis or chemical synthesis. Moreover, the construct can be prepared in such a way that an organic compound that acts as a nucleic acid such as various types of DNA or RNA, a dye (for example, various types of fluorescent compounds such as FAM and FITC), or a drug (for example, an antitumor drug including a nucleic acid-based antitumor drug such as 5-fluorouracil (5FU), an antiviral drug such as azidothymidine (AZT), etc.) is directly or indirectly bonded to the N-terminus and/or the C-terminus of the above carrier peptide fragment by various publicly known scientific methods.

Although there is no particular limitation, the function of the foreign substance can be, for example, promoting differentiation induction of stem cells (stem cell differentiation inducing activity), growth inhibition of tumor cells (antitumor activity), growth inhibition of virus-infected cells (antivirus activity), or the like.

In the construct disclosed herein, the number of foreign substances to be bonded to the carrier peptide fragment is not limited in particular. For example, one or more foreign substances may be bonded to one carrier peptide fragment. Although there is no particular limitation, for example, a polypeptide, a nucleic acid, a drug, or the like may be bonded to the C-terminus of one carrier peptide fragment and then a dye may be bonded to the N-terminus thereof. It is preferable to bond the dye to the carrier peptide fragment because it becomes easy to evaluate the efficiency of transferring the construct into the eukaryotic cell and the localization in the cell.

Note that when the foreign substance is a polypeptide, the polypeptide (amino acid sequence) to be used is not particularly limited. Proteins or a polypeptide with a relatively large number of amino acid residues, for example about 100 to 1000 amino acid residues, can also be used as the foreign substance.

Typically, the total number of amino acid residues constituting the synthetic peptide manufactured as the construct for transferring a foreign substance is several to several tens (for example, 10) or more and suitably 1000 or less, preferably 600 or less, more preferably 500 or less, and particularly preferably 300 or less (for example, 10 to 300). The polypeptide with such a length is easy to synthesize (biosynthesis, chemical synthesis) and easy to use.

Preferably, the foreign substance to be used is a mature form or precursor (including pro-forms and prepro-forms) of a polypeptide involved in a function such as the development, differentiation, growth, malignant transformation, homeostasis, or regulation of metabolism in various cells and tissues (organs). Moreover, the method for transferring a foreign substance disclosed herein can be carried out to transfer a polypeptide with a heretofore unknown function into a cell to elucidate the function of the polypeptide in the cell (in a biological tissue).

For example, if the eukaryotic cell that is the target to which the foreign substance is to be transferred is a human or other mammalian stem cell, it is preferable to use a mature form or precursor of a polypeptide with various biological activities related to the differentiation induction of the stem cells. Note that the term "stem cell" encompasses somatic stem cells, embryonic stem cells, and induced pluripotent stem cells (hereinafter, iPS cells). Moreover, when the eukaryotic cell to which the foreign substance is to be transferred is a cancer cell (tumor cell), it is preferable to use various polypeptides involved in the induction of apoptosis of the cancer cell (tumor cell). Alternatively, in this case, it is preferable to use a polypeptide that can prevent a cancer cell (tumor cell) from inhibiting the function of an immunity monitoring mechanism. Moreover, when the eukaryotic cell that is the target of transfer is a bacteria-infected cell or a virus-infected cell, it is preferable to use various polypeptides involved in inducing apoptosis of these infected cells, a polypeptide that can inhibit the increase of bacteria or virus in the infected cells, or a polypeptide that can inhibit the expansion of the bacteria or virus infection from the infected cells.

Furthermore, similarly to the carrier peptide fragment, the polypeptide as the foreign substance may include a modified amino acid sequence that is formed by the replacement, deletion, and/or addition (insertion) of one or several amino acid residues as long as that function is kept.

In the construct in which the foreign substance is bonded to the C-terminus of the carrier peptide fragment, an α-amino group of the amino acid residue of the N-terminus of the carrier peptide fragment is preferably acetylated. Although the detailed mechanism is not clear, since the α-amino group of the amino acid of the N-terminus in most of the proteins in the eukaryotic cell is subjected to the acetylation modification, such a structure can improve the stability of the construct in the cells.

In the construct, the amino acid residue of the C-terminus is preferably amidated. The structural stability (for example, protease resistance) of the construct as described above in the cytoplasm and nucleolus can be improved by amidation of a carboxyl group of the amino acid residue (typically, a C-terminal amino acid residue of a peptide chain). In addition, the amidation of the carboxyl group improves the hydrophilicity of the construct, so that the solubility of this construct in an aqueous solvent can be improved. Examples of such an aqueous solvent include water, various buffer solutions, physiological saline (for example, PBS), a cell culture solution, and the like.

For example, in the case of the construct in which the foreign substance is bonded to the N-terminus of the carrier peptide fragment, a carboxyl group of the amino acid residue of the C-terminus of the carrier peptide fragment is preferably amidated. In a case where, for example, the foreign substance is a polypeptide and this polypeptide is bonded to the C-terminus of the carrier peptide fragment, it is preferable to amidate the carboxyl group of the C-terminal amino acid residue of the polypeptide.

Among constructs, those with a relatively short peptide chain (encompassing a polypeptide, a carrier peptide fragment, and a peptidic linker configured as a foreign substance) can easily be manufactured by a general chemical synthesis method. For example, either a conventionally known solid phase or liquid phase synthesis method can be used. A solid phase synthesis method using Boc (t-butyloxycarbonyl) or Fmoc (9-fluoroenylmethoxycarbonyl) as a protecting group for an amino group is preferred. In other words, the aforementioned peptide chain with the desired amino acid sequence and modifications (N-terminal acetylation, C-terminal amidation, etc.) can be synthesized by a solid phase synthesis method using a commercially available peptide synthesizer. Note that only a part of the peptide chain may be synthesized by the aforementioned method and for example, the peptide chain including only the carrier peptide fragment or including the carrier peptide fragment and the peptidic linker can be synthesized.

Alternatively, the peptide part may be manufactured by biosynthesis in accordance with the genetic engineering method. In other words, a polynucleotide (typically, DNA) of a nucleotide sequence (including the ATG start codon) that codes for the desired amino acid sequence is synthesized. Then, a recombinant vector with a genetic construct for expression that includes the synthesized polynucleotide (DNA) and various regulatory elements (including a promoter, ribosome binding site, terminator, enhancer, and various cis-elements for controlling the level of expression) to express the amino acid sequence in a host cell is configured in accordance with the host cell.

Using the general techniques, this recombinant vector is transferred to a predetermined host cell (for example, yeast, insect cell, or plant cell), and the host cell, or an individual or tissue containing the cell is cultured under a predetermined condition. The target peptide can be produced in the cell thereby. Furthermore, the target peptide part can be obtained by isolating the peptide part from the host cell (or from a culture medium if it is secreted) and if necessary, refolding and purifying the peptide part, for example.

Note that the method for configuring the recombinant vector and the method for transferring the configured recombinant vector to the host cell, etc., may utilize methods conventionally used in the fields without modification, and because those methods themselves are not a characterizing feature of the present art, the detailed explanation thereof is omitted.

For example, a fusion protein expression system can be used for efficient, large volume production in host cells. More specifically, first, the gene (DNA) coding for the amino acid sequence of the target polypeptide is obtained by chemical synthesis, and the synthesized gene is transferred to a suitable site in a suitable fusion protein expression vector (for example, a glutathione S-transferase (GST) fusion protein expression vector such as the pET series provided by Novagen and the pGEX series provided by Amersham Biosciences). Then, the host cells (typically E. coli) are transformed by the vector. The resulting transformant is cultured to prepare the target fusion protein. Next, the protein is extracted and purified. Then, the resulting purified fusion protein is cleaved by a predetermined enzyme (protease) and the freed target peptide fragment (i.e., the designed artificial polypeptide) is recovered by a method such as affinity chromatography. The target construct (artificial polypeptide) can be manufactured using this kind of conventionally known fusion protein expression system (for example, the GST/His system provided by Amersham Biosciences can be utilized).

Alternatively, template DNA for use in a cell-free protein synthesis system (i.e., a synthetic gene fragment containing a nucleotide sequence coding for the amino acid sequence of the peptide part for the construct) can be configured, and in-vitro synthesis of the target polypeptide can be carried out by employing a so-called cell-free protein synthesis system using various compounds necessary for synthesis of the peptide part (ATP, RNA polymerase, amino acids, etc.). References concerning a cell-free protein synthesis system include, for example, the papers by Shimizu et al. (Shimizu et al., Nature Biotechnology, 19, 751-755 (2001)) and Madin et al. (Madin et al., Proc. Natl. Acad. Sci. USA, 97(2), 559-564 (2000)). At the time of the filing of the present application, there are already many companies carrying out polypeptide production on consignment on the basis of the technology disclosed in these documents, and cell-free protein synthesis kits are commercially available (for example, obtainable from Cell Free Sciences Co., Ltd. in Japan).

The nucleotide sequence coding for the peptide part of the construct and/or a single-chain or double-chain polynucleotide including the nucleotide sequence that is complementary to the above-described nucleotide sequence can be manufactured (synthesized) easily by the conventionally known method. That is to say, by selecting the codon that corresponds to each amino acid residue constituting the designed amino acid sequence, the nucleotide sequence corresponding to such an amino acid sequence is easily determined and provided. Once the nucleotide sequence is determined, the polynucleotide (single chain) corresponding to the desired nucleotide sequence can be easily obtained using a DNA synthesizer or the like. Furthermore, by using the obtained single-chain DNA as a template, the target double-chain DNA can be obtained with various enzymatic synthesizing means (typically, PCR). Moreover, the polynucleotide may be either in a DNA form or an RNA (mRNA, etc.) form. DNA can be provided as a double chain or a single chain. In the case of providing DNA as a single chain, the chain may be either a code chain (sense chain) or a non-code chain (anti-sense chain) with the sequence complementary to that code chain.

The polynucleotide obtained in this manner can be used as a material for configuring a recombination gene (expression cassette) for peptide production in various host cells or a cell-free protein synthesis system as described above.

The construct disclosed herein can be suitably used as an effective component of the composition for the usage based on the function of the foreign substance. Note that the construct may be in a salt form unless the function of the foreign substance is lost. For example, an acid addition salt that can be obtained by carrying out an addition reaction with a normally used inorganic or organic acid in accordance with a conventional method can be used. Thus, the "construct" according to the present specification and the scope of claims can encompass the construct in such a salt form.

The construct can be used as an effective component of the composition that can contain various carriers that are allowable in terms of medical (pharmaceutic) perspectives in accordance with the usage mode.

As the carrier, for example, a carrier that is generally used in the peptide medical fields such as a diluent or an excipient is preferable. As such a carrier, typically, water, a physiological buffer, and various organic solvents are given, although depending as appropriate on the usage or mode of the construct for transferring a foreign substance. Moreover, the carrier can be an alcohol (such as ethanol) aqueous solution with a suitable concentration, glycerol, or non-drying oil such as olive oil, or may be liposome. As a secondary component that can be contained in a pharmaceutical composition, various filling agents, thickening agents, bonding agents, moisture adding agents, surfactants, dyes, flavoring agents, and the like are given.

The mode of the composition is not limited in particular. For example, modes of liquids, suspensions, emulsions, aerosols, foams, granules, powders, tablets, capsules, and ointments are given. Moreover, for the use in injection or the like, a lyophilized product or granulated product to prepare a drug solution by being dissolved in physiological saline or a suitable buffer (e.g., PBS), etc., just before use can be formed.

The conventionally known methods may be used for the processes themselves to prepare various modes of medicines (compositions) from the construct (main component) and various carriers (secondary components) as materials, and a detailed explanation of such a manufacturing process itself is omitted herein because it is not a characterizing feature of the present art. For example, Comprehensive Medicinal Chemistry, edited by Corwin Hansch, Pergamon Press, 1990, can be noted as a source of detailed information concerning formulations.

The present disclosure provides a method for transferring a foreign substance in vivo or in vitro using the construct disclosed herein. This method includes the following steps (1) and (2):
(1) a step of preparing a construct disclosed herein; and
(2) a step of supplying the construct into a sample including a target eukaryotic cell.
The method disclosed herein can further include, after the step (2), a step (3) of incubating the sample to which the construct has been supplied, thereby transferring the construct into the eukaryotic cell in the sample.

The term "eukaryotic cell" encompasses, in vivo, various tissues, viscera, organs, blood, lymph, and the like, for example. The term "eukaryotic cell" encompasses, in vitro, various cell clusters, tissues, viscera, organs, blood, lymph, cell lines, and the like extracted from a body, for example. Examples of the eukaryotic cell include cells derived from Fungi, Plantae, Animalia such as mammals, birds, fishes, amphibians, reptiles, and insects, and the like. Preferably, the eukaryotic cell can be a human or nonhuman mammalian cell.

The composition including the construct disclosed herein can be used in vivo in dosage and method according to the mode and intended purpose. For example, exactly a desired amount of liquid can be administered to a diseased area (for example, malignant tumor tissue, virus-infected tissue, inflamed tissue, etc.) of a patient (i.e., the body) by intravenous, intramuscular, subdermal, intradermal, or intraperitoneal injection. Alternatively, the composition in a solid mode such as a tablet or in a gel-form or aqueous jelly-form such as ointment can be applied directly to a predetermined tissue (i.e., for example, a diseased area such as an organ or a tissue including a tumor cell, virus-infected cell, inflamed cell, or the like). Further alternatively, the composition in a solid mode such as a tablet can be administered orally. In the case of the oral administration, it is preferable to perform encapsulation or apply a protection (coating) material in order to suppress decomposition by digestive enzymes in the alimentary canal.

Alternatively, the construct in an amount suitable for the eukaryotic cell cultured in vitro is preferably supplied to a culture solution of the target eukaryotic cell at least once. The amount and the number of times per supply are not limited in particular because they can vary depending on the conditions including the kind of eukaryotic cells to culture, the cell density (cell density at the start of culture), the passage number, the culture condition, the kind of culture medium, and the like. For example, the composition is preferably added once, twice, or more times so that the concentration of the carrier peptide fragment in the culture solution falls within the range of about 0.05 µM or more and 100 µM or less, 0.5 µM or more and 50 µM or less, or 1 µM or more and 30 µM or less, for example. In addition, the incubating time after the construct is added is not limited in particular either because the incubating time can vary depending on the kind of eukaryotic cells and various conditions. For example, the incubating time can be 0.5 hours or more, 1 hour or more, 4 hours or more, 8 hours or more, or 20 hours or more. Moreover, the incubating condition is not limited in particular because the incubating condition can vary depending on the kind of eukaryotic cells; however, the incubation is possible in a 5% CO₂ atmosphere under 37°C, for example.

Note that one example of the transferring method in vitro will be described in test examples below.

A method for evaluating the transferring efficiency of the construct is not limited in particular. For example, in the case where a dye (typically, a fluorescent compound) is bonded to the construct, it is possible to evaluate the transferring efficiency into the eukaryotic cells by using microscope observation (for example, fluorescence microscope observation), flow cytometry, or the like. Alternatively, the transferring efficiency of the construct can be evaluated by an immunochemical method (for example, Western Blot, immunocytochemistry, or the like) that uses an antibody to recognize the peptide part of the construct specifically.

As described above, the following items are given as specific aspects of the art disclosed herein.
Item 1: The carrier peptide fragment including the following amino acid sequence set forth in SEQ ID No.: 1: KKRTLRKSNRKKR, in which the lysine residue at the 12th position from the N-terminus of the amino acid sequence is the D-form, and the other amino acid residues are the L-forms.
Item 2: The construct including the carrier peptide fragment according to Item 1, and the foreign substance that is bonded to the N-terminus and/or the C-terminus of the carrier peptide fragment.
Item 3: The construct according to Item 2, in which the foreign substance is at least one kind of organic compound selected from the group consisting of a polypeptide, a nucleic acid, a dye, and a drug.
Item 4: The construct according to Item 2 or 3, in which the foreign substance is disposed on the C-terminus of the carrier peptide fragment.
Item 5: The method for transferring a target foreign substance in vitro from outside the eukaryotic cell into at least the cytoplasm of the cell, the method including the steps of: (1) preparing the construct according to any one of Items 2 to 4; and (2) supplying the construct into the sample including the target eukaryotic cell.
Item 6: The method according to Item 5, in which the eukaryotic cell to which the construct is to be transferred is the human or nonhuman mammalian cell.

Several test examples concerning the art disclosed herein are described below, but it is not intended to limit the art disclosed herein to these test examples.

### <Manufacture of construct for transferring foreign substance>

Constructs including synthetic peptides with amino acid sequences shown in Table 1 were prepared. A construct including a peptide n (n is a natural number of 1 to 15) shown in Table 1 is a sample n, and samples 1 to 15 were obtained from Eurofins Genomics K.K. Note that in the amino acid sequences shown in Table 1, the amino acid residue represented in a capital letter corresponds to the L-form, and the amino acid residue represented in a small letter corresponds to the D-form. In the prepared samples 1 to 15, the α-amino group of the amino acid residue of the N-terminus in the peptides 1 to 15 were acetylated. In addition, FAM (C₂₁H₁₂O₇: 5(6)-carboxyfluorescein, molecular weight 376.3, excitation wavelength 495 nm, fluorescence wavelength 520 nm) corresponding to fluorescent dye was bonded as the foreign substance to the amino acid residue of the C-terminus of each of the peptides 1 to 15 in the preparation. The samples 1 to 15 were each diluted in dimethyl sulfoxide (DMSO) to prepare sample solutions 1 to 15 with a sample concentration of 2 mM.

**[Table 1]**

| Table 1 | | |
|---|---|---|
| peptide No. | Sequence | SEQ ID No. |
| 1 | kKRTLRKSNRKKR | 3 |
| 2 | KkRTLRKSNRKKR | 4 |
| 3 | KKrTLRKSNRKKR | 5 |
| 4 | KKRtLRKSNRKKR | 6 |
| 5 | KKRTlRKSNRKKR | 7 |
| 6 | KKRTLrKSNRKKR | 8 |
| 7 | KKRTLRkSNRKKR | 9 |
| 8 | KKRTLRKsNRKKR | 10 |
| 9 | KKRTLRKSnRKKR | 11 |
| 10 | KKRTLRKSNrKKR | 12 |
| 11 | KKRTLRKSNRkKR | 13 |
| 12 | KKRTLRKSNRKkR | 1 |
| 13 | KKRTLRKSNRKKr | 14 |
| 14 | kkrtlrksnrkkr | 15 |
| 15 | KKRTLRKSNRKKR | 2 |

### <Evaluation of cell membrane permeability by flow cytometry>

As the eukaryotic cells, HeLa cells (established cell strain derived from a human cervical cancer cell) were used and the cell membrane permeability of the samples 1 to 15 was analyzed. In Examples 1 to 15, the prepared sample solutions 1 to 15 were used, respectively, and in Example 16, the FAM solution was used.

### (Example 1)

The HeLa cell was cultured in 10% fetal bovine serum (FBS)-containing Dulbecco's modified Eagle's medium (DMEM (Cat No. 044-29765, manufactured by FUJIFILM Wako Pure Chemical Corporation)), which is a culture medium.

The HeLa cell that adhered to a culture plate was cleaned with PBS and then, a 0.25%-trypsin/EDTA solution was added thereto, and incubation was performed for three minutes at 37°C. After the incubation, the aforementioned 10% FBS-containing DMEM was added to deactivate trypsin and then, five-minute centrifugal separation was performed at 150 × g to precipitate the cells. After the supernatant generated by the centrifugal separation was removed, the 10% FBS-containing DMEM was added to the precipitate (cell pellet) to prepare a cell suspension of about 2 × 10⁵ cells/mL. The cell suspension was added by 1 mL to a well of a commercially available six-hole (well) plate (manufactured by AGC TECHNO GLASS Co., Ltd.) so as to seed the cell (about 2 × 10⁵ cells/well). In addition, the 2 mM sample solution 1 was diluted with the 10% FBS-containing DMEM to prepare the sample solution 1 in which the concentration of the sample 1 was 20 µM. Then, 1 mL of the 20 µM sample solution 1 was added to the well (that is, so that the concentration of the sample 1 became 10 µM and the DMSO concentration became 0.5% in the culture solution in the well). After that, the cell was incubated for 20 hours at 37°C under a 5% CO₂ condition.

After the 20-hour incubation, the culture supernatant was removed from the well and the cells in the well were cleaned twice with 1 mL of PBS. Next, 200 µL of the 0.25% trypsin/EDTA solution was added to the well and the incubation was performed for three minutes at 37°C. After the incubation, 400 µL of the 10% FBS-containing DMEM was added to the well to deactivate the trypsin. Subsequently, the cell suspension in the well was transferred to a tube and the cells were collected. After that, 600 µL of PBS was added further to the well to clean the well. Then, PBS in the well was transferred to the tube, so that the cells remaining in the well were collected in the tube. This tube was subjected to five-minute centrifugal separation at 4°C under a condition of 210 × g. After the centrifugal separation, the supernatant was removed and the precipitate (cell pellet) was suspended (cleaned) with 1 mL of PBS, which was followed by the centrifugal separation under the same condition as that described above. After this operation was repeated twice, the supernatant was removed and thus, the cells (cell pellet) cultured in a culture medium containing the sample 1 were obtained.

Regarding the obtained cells (cell pellet), the cell membrane permeability of the sample 1 was analyzed using a flow cytometer. As the flow cytometer, On-Chip Flowcytometer (manufactured by On-Chip Biotechnologies Co., LTD.) was used.

For such an analysis, the obtained cell pellet was suspended with 100 µL of on-chip T buffer to prepare a cell suspension for analysis.

Using the aforementioned flow cytometer, gating based on forward scatter (FSC) and side scatter (SSC) was performed to set a gate about a cell group to be analyzed, and the fluorescence intensity was measured about the cell group in this gate. Note that the analysis was performed so that the cell group contained at least 10000 cells. The fluorescence intensity was measured using a fluorescence detector FL2 of the flow cytometer (optimum detection wavelength around 543 nm) capable of detecting the fluorescence wavelength of FAM. Regarding this measurement result, the analysis was performed using commercially available analysis software "FlowJo (registered trademark)" (manufactured by TreeStar) to obtain median fluorescence intensity (MFI) of the cell group to be measured.

### (Examples 2 to 15)

The process similar to that in Example 1 was performed except that any of the prepared sample solutions 2 to 15 was used instead of the sample solution 1. Note that the samples (constructs) used in the respective examples are as shown in Table 2.

### (Example 16)

The process similar to that in Example 1 was performed except that the FAM solution diluted with DMSO was used instead of the sample solution 1. The concentration of this FAM solution was the same as the concentration of the sample solution 1 (that is, the FAM concentration was 10 µM and the DMSO concentration was 0.5% in the culture solution in the well).

The results obtained from Examples 1 to 16 are shown in Table 2 and Fig. 1. Fig. 1 is a graph illustrating the values of MFI in each example.

### [Table 2]

**Table 2**

| | Structure of construct (additive) | MFI |
|---|---|---|
| Example 1 | Ac-kKRTLRKSNRKKR-FAM | 13.9 |
| Example 2 | Ac-KkRTLRKSNRKKR-FAM | 14.1 |
| Example 3 | Ac-KKrTLRKSNRKKR-FAM | 13.9 |
| Example 4 | Ac-KKRtLRKSNRKKR-FAM | 15.9 |
| Example 5 | Ac-KKRTIRKSNRKKR-FAM | 16.5 |
| Example 6 | Ac-KKRTLrKSNRKKR-FAM | 17.5 |
| Example 7 | Ac-KKRTLRkSNRKKR-FAM | 16.9 |
| Example 8 | Ac-KKRTLRKsNRKKR-FAM | 14.3 |
| Example 9 | Ac-KKRTLRKSnRKKR-FAM | 15.6 |
| Example 10 | Ac-KKRTLRKSNrKKR-FAM | 15.5 |
| Example 11 | Ac-KKRTLRKSNRkKR-FAM | 17.4 |
| Example 12 | Ac-KKRTLRKSNRKkR-FAM | 33.8 |
| Example 13 | Ac-KKRTLRKSNRKKr-FAM | 15.2 |
| Example 14 | Ac-kkrtirksnrkkr-FAM | 16.1 |
| Example 15 | Ac-KKRTLRKSNRKKR-FAM | 13.0 |
| Example 16 | FAM | 10.6 |

As shown in Table 2 and Fig. 1, the value of MFI was higher in Examples 1 to 14 than in Example 15. Therefore, it is found that when at least one of amino acid residues in an amino acid sequence set forth in SEQ ID No.: 2 is the D-form amino acid residue, a cell membrane permeation rate is improved. Moreover, as shown in Table 2 and Fig. 1, the value of MFI was high in particular in Example 12. Thus, it is found that when only the lysine residue at the 12th position from the N-terminus of the amino acid sequence: KKRTLRKSNRKKR is the D-form, the cell membrane permeability is remarkably improved.

The specific examples of the art disclosed herein have been described above in detail; however, these are just examples and will not limit the scope of claims. The art described in the scope of claims includes those in which the specific examples given above are variously modified and changed.

The art disclosed herein provides the carrier peptide fragment and the construct including the carrier peptide fragment that are artificially manufactured for transferring a target foreign substance from outside the eukaryotic cell (in particular, various animal cells typified by human and nonhuman mammalian cells that do not have a cell wall) into the cytoplasm thereof. By utilizing this construct, the target foreign substance can be effectively transferred into the target cell, and biological tissues such as organs including cells to which the foreign substance has been transferred and cells that contain the foreign substance can be obtained. In addition, by utilizing the carrier peptide fragment or the construct, therapeutic agents for various diseases (for example, therapeutic agents for ophthalmic treatment and the like, and oligonucleotide therapeutics) can be provided. In addition, the peptide fragment or the construct disclosed herein can be used as an active ingredient or an additive in medicine for external use (for example, eye drops).

## Claims

1. A carrier peptide fragment comprising the following amino acid sequence set forth in SEQ ID No.: 1:
KKRTLRKSNRKKR, wherein
a lysine residue at a 12th position from an N-terminus of the amino acid sequence is a D-form, and the other amino acid residues are L-forms.

2. A construct comprising:
the carrier peptide fragment according to claim 1; and
a foreign substance that is bonded to the N-terminus and/or a C-terminus of the carrier peptide fragment.

3. The construct according to claim 2, wherein the foreign substance is at least one kind of organic compound selected from the group consisting of a polypeptide, a nucleic acid, a dye, and a drug.

4. The construct according to claim 2, wherein the foreign substance is disposed on the C-terminus of the carrier peptide fragment.

5. A method for transferring a target foreign substance in vitro from outside a eukaryotic cell into at least a cytoplasm of the cell, the method comprising the steps of:
(1) preparing the construct according to any one of claims 2 to 4; and
(2) supplying the construct into a sample including a target eukaryotic cell.

6. The method according to claim 5, wherein the eukaryotic cell to which the construct is to be transferred is a human or nonhuman mammalian cell.
